(19) **Europäisches Patentamt** / **European Patent Office** / **Office européen des brevets**

(11) **EP 2 066 709 B1**

(12) **EUROPEAN PATENT SPECIFICATION**

(45) Date of publication and mention
of the grant of the patent:
**11.04.2012 Bulletin 2012/15**

(51) Int Cl.:
*C08J 3/12* (2006.01)      *C08J 9/36* (2006.01)
*G01N 33/543* (2006.01)   *G01N 33/53* (2006.01)
*G01N 33/68* (2006.01)     *C12N 15/10* (2006.01)
*H01F 1/06* (2006.01)       *C12Q 1/68* (2006.01)

(21) Application number: **07865890.3**

(22) Date of filing: **19.12.2007**

(86) International application number:
**PCT/US2007/088235**

(87) International publication number:
**WO 2008/079905 (03.07.2008 Gazette 2008/27)**

(54) **PARTICLES AND THEIR USE IN A METHOD FOR ISOLATING NUCLEIC ACID OR A METHOD FOR ISOLATING PHOSPHOPROTEINS**

PARTIKEL UND IHRE VERWENDUNG IN EINEM VERFAHREN ZUR NUKLEINSÄUREISOLIERUNG ODER EINEM VERFAHREN ZUR PHOSPHORPROTEINISOLIERUNG

PARTICULES ET LEUR UTILISATION DANS UN PROCÉDÉ D'ISOLEMENT D'ACIDE NUCLÉIQUE OU DE PHOSPHOPROTÉINES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.12.2006 GB 0625652**
**21.12.2006 US 871443 P**
**21.12.2006 US 871429 P**
**21.12.2006 GB 0625650**
**21.12.2006 GB 0625626**
**21.12.2006 US 871440 P**

(43) Date of publication of application:
**10.06.2009 Bulletin 2009/24**

(73) Proprietors:
• **Invitrogen Dynal AS**
**0309 Oslo (NO)**
• **University of Southern Denmark**
**5230 Odense M (DK)**

(72) Inventors:
• **FONNUM, Geir**
**N-1472 Fjellhamar (NO)**
• **STENE, Torkel**
**N-0682 Oslo (NO)**
• **KJUS, Nini, Hofsloekken**
**N-0679 Oslo (NO)**
• **BERGHOLTZ, Stine**
**N-0480 Oslo (NO)**
• **POPE, Robert, Marshall**
**San Marcos, CA 92069 (US)**
• **LIANG, Xiquan**
**Escondido, CA 92026 (US)**
• **KIRPEKAR, Finn**
**DK-5230 Odense M (DK)**
• **ENGHOLM-KELLER, Kasper**
**DK-5230 Odense M (DK)**
• **LARSEN, Martin, R.**
**DK-5230 Odense M (DK)**
• **JENSEN, Ole, N.**
**DK-5230 Odense M. (DK)**

(74) Representative: **Weber, Birgit**
**Life Technologies GmbH**
**Frankfurter Strasse 129 B**
**64293 Darmstadt (DE)**

(56) References cited:
**US-A- 4 774 265      US-A- 5 091 206**
**US-A- 5 814 687      US-B2- 6 797 782**

• **GUO H.-X. ET AL.: 'The synthesis of composite particles responsive to electric and magnetic fields' OPTICAL MATERIALS vol. 22, March 2003, pages 39 - 44, XP004402288**

- CHEN C.-T. ET AL.: 'Fe3O4/TiO2 core/shell nanoparticles as affinity probes for the analysis of phosphopeptides using TiO2 surface-assisted laser deposition/ionization mass spectrometry' ANALYTICAL CHEMISTRY vol. 77, no. 18, September 2005, pages 5912 - 5919, XP009090888

**Description**

This invention relates to magnetic polymer particles coated with a transition metal oxide, in particular titanium and zirconium oxides and to processes for the production thereof. The invention further relates to use of the coated magnetic polymer particles in combination with specific buffers for purifications, extractions, captures, assays or syntheses.

Magnetic particles are of general utility in various medical, biochemical and environmental fields, for example as transport vehicles for the delivery of pharmaceutical products, for diagnostic or analytical purposes, for separation of analytes and for synthetic purposes. Such particles rely upon their magnetic properties in order to perform these functions. For example, in assays application of a magnetic field to a sample containing, *inter alia,* particle-bound analyte enables isolation of the analyte without the use of centrifugation or filtration.

By magnetic is meant herein that the polymeric particles are capable of being attracted by a magnetic field. Typically such polymeric particles contain ferromagnetic crystals, superparamagnetic crystals or a mixture thereof.

Magnetic polymer particles are known and may, for example, be prepared according to the processes described US-A-4,654,267 (Ugelstad). These processes provide magnetic polymer particles that are of similar size (monodisperse) and which possess homogeneous magnetic properties.

The surface of these particles can be readily functionalised to carry various groups which can be used to bind affinity ligands for analytes in a sample. For example, beads modified to carry streptavidin are known. The person skilled in the art is however, looking for further ways to modify polymer particles to allow their use in a variety of rapid non-ligand based adsorption procedures,

In order to minimise the known problem of leaching of magnetic crystals from the polymer particles, which shortens their lifetime and potentially contaminates samples, US-A-4,654,267 advocates the use of polymer particles having surface functional groups which serve to draw the Fe ions into the polymeric particles. These functional groups could either result from the use of functionalized co-monomers in the production of the polymer particles or from post-polymerization treatment of the polymer particle to introduce the functional groups, e.g. by coupling to or transformation of existing groups on the particle surface.

Whilst the invention disclosed in US-A-4,654,267 does decrease the amount of leaching, some leaching of the superparamagnetic crystals from the polymer particles still occurs. Moreover the use of particular surface functional groups to draw in the Fe ions places significant limitations on the nature of the surface functionality that can be provided on the polymer particles. This in turn makes further functionalization of the particles (e.g. with ligands) more difficult. Thus, the versatility of the particles is decreased and the range of applications in which the polymer particles may be used is restricted.

We have now surprisingly discovered that the problem of leaching as well as the provision of an alternatively functionalised polymer particle is provided by optionally porous, optionally magnetic polymer microparticles having a coating formed from at least one transition metal oxide. Not only does the presence of a transition metal oxide coating minimise the potential problem of leaching, the use of a transition metal oxide coating allows the polymer particle to bind readily to biopolymers making the particles exceedingly valuable in assay procedures.

Magnetic particles with metal oxide coatings are not in themselves new. In Anal. Chem 2005, 77,5912-5919, iron (III) oxide/titania core shell nanoparticles are described and suggested for use as affinity probes for the analysis of phosphopeptides.

Also, the use of metal oxides to bind nucleic acid is not new. US 6,383,393 describes a method for purification and separation of nucleic acid mixtures by chromatography. The mixture of nucleic acids is adsorbed on a metal oxide substrate on a column. US 5,057,426 has a similar disclosure were a metal oxide matrix is used to bind nucleic acid. These disclosures are however, in the context of chromatographic columns and are not at all related to polymer particles.

US 6,914,137 describes a method of DNA isolation using polystyrene microtitre plates containing titanium oxide which was incorporated as a powder in the plastic when the plate was formed.

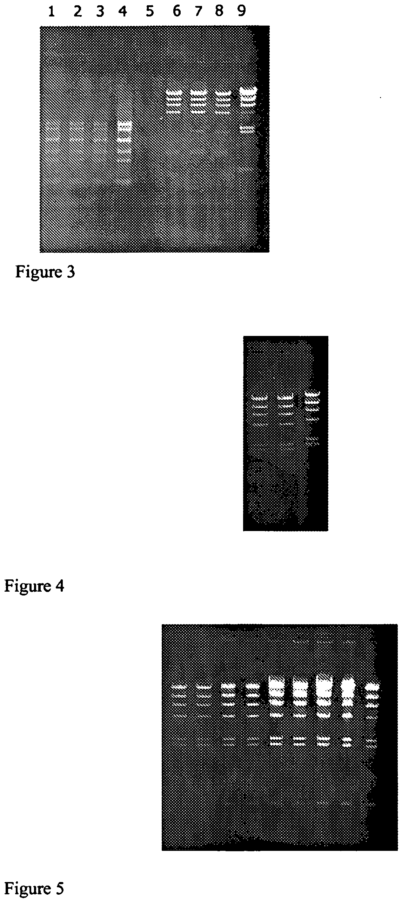

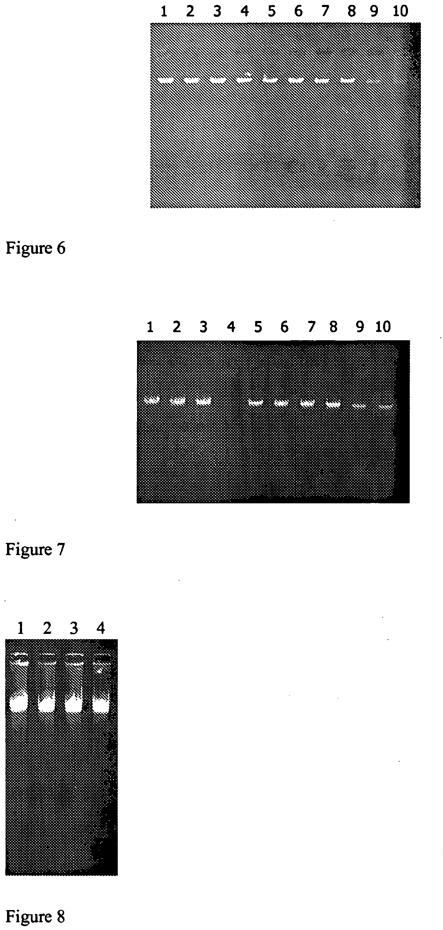

Particles coated with titanium oxide are also known. Guo et al in Optical Materials 22 (2003), 39-44 describes core-shell particles in which a dense polystyrene, iron (III) oxide core is coated with titania. These particles differ considerably however, from the magnetic porous particles of this invention. The polystyrene/iron cores in Guo are dense, non porous materials and thus the problem of leaching is not one which is faced by Guo. Moreover, no suggestion of the use of such particles in the isolation of biomolecules is given. The IR spectra of Guo et al's particles (their Figure 3), shows that they contain very little iron. This is also indicated in their Figure 7 where the authors show that they have about 90% recovery of particles after 15 minutes incubation on a magnet.

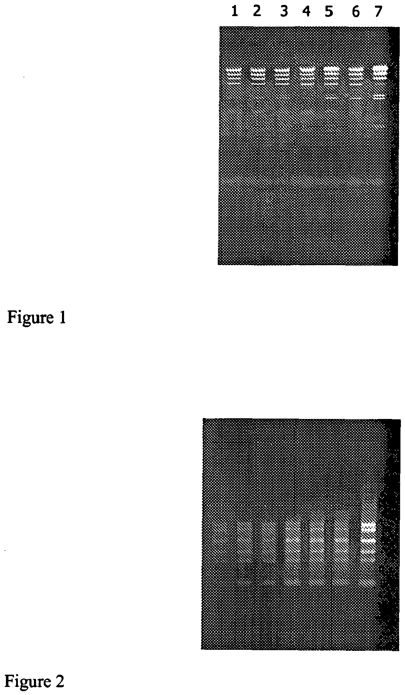

Furthermore, as seen in their Figure 2, the particles have a wide particle size distribution and are not therefore monodisperse. The use of such particles for isolation of biopolymers is therefore unfavourable as much of the target will be lost as it will bind to the smaller particles which will not be attracted to the magnet even after 15 min. incubation time. Also, as seen from their figure 1, the particles are highly aggregated, again making them unsuitable for bioseparation.

Hence, viewed from a first aspect the present invention provides monodisperse polymer microparticles comprising polystyrene or polyacrylate, wherein said particles have a coating formed of at least one transition metal oxide.

Viewed from another aspect the present invention provides porous polymer microparticles comprising polystyrene or polyacrylate, wherein said particles have a coating formed of at least one transition metal oxide.

Viewed from a second aspect, the present invention provides a process for the preparation of particles as hereinbefore described comprising reacting monodisperse and porous polymer microparticles comprising polystyrene or polyacrylate with at least one transition metal compound capable of being converted into an oxide and forming the transition metal oxide coating, e.g. upon application of heat and/or water.

Viewed from a further aspect, the invention provides the use of these particles in biopolymer isolation.

The polymer particles of the present invention comprise a core polymer seed which is swelled using the well known Ugelstad type process. In this application, the words bead and particle are used interchangeably. The core polymer may be a polyacrylate but is preferably formed from polystyrene and thus the seed material is typically a polystyrene particle of the order of 0.1 to 0.5 microns in diameter. As is well known in the art, such a core can be swelled by allowing monomer (and normally a polymerisation initiator) to diffuse into the core prior to polymerisation being initiated. A variety of monomers can be used during this swelling process. Preferred monomers include styrene or acrylate monomers, e.g. methacrylates.

The particles of the invention are preferably porous. The particles are preferably made porous by using porogens during the manufacturing process as is known in the art. Moreover, it is known to use steric stabilisation, particular surfactants, oxygen free conditions, etc in polymer particle manufacture.

The particles of use in this invention can be made by a process in which monomer is pre-swelled into the core particle before polymerisation or a process in which after a short initial monomer swell period, polymerisation is initiated whilst further monomer is added.

The polymer formed during the swelling stage of particle formation may be a polystyrene homopolymer or an acrylate polymer (e.g. of methacrylate, glycidyl methacrylate and ethyleneglycol dimethacrylate) but is more preferably a copolymer of styrene. Copolymers may be formed with any co-monomer capable of copolymerising with styrene, e.g. divinylbenzene (DVB), amino-styrene, and nitro-styrene, especially divinylbenzene.

Preferably therefore, this polymer is cross-linked, for example, by incorporation of cross linking agents, e.g. as co-monomers. Suitable cross linking agents include divinylbenzene (DVB) or ethyleneglycol dimethacrylate. DVB is preferred. Appropriate quantities of cross-linking agents (e.g. co-monomers) required will be well known to the skilled man but typically will be 30 to 85 % (by wt relative to the weight of monomer), e.g. about 50 % wt. Preferably therefore, the polymer particles of use in the invention are cross-linked styrenic polymer particles (e.g. a styrene-divinylbenzene polymer particle).

Prior to coating with the transition metal compound, the polymeric particles of the present invention are preferably surface-functionalised. In other words the surface of the polymeric particles is preferably provided with a group capable, optionally with activation, of reacting or interacting with a transition metal compound (e.g. to covalently bond the compound to the surface). Preferably the surface is amine functionalised, e.g. by direct amination with ethylene diamine or by nitrating the surface and reducing the nitro groups to amines. Such an amino functionalised surface provides an ideal template for direct reaction with the transition metal compound or for further functionalisation prior to reaction with the transition metal compound. For example, the surface can be functionalised as described in WO05/015216 by reaction with epoxides or diols/isocyanates. A hydroxy functionalised surface or polyurethane functionalised surface may result. By carefully selecting the nature of the epoxide monomer, a surface carrying vinyl groups can also be formed.

Polymeric particles having multiple coatings have been found to exhibit even less leaching than their monolayered counterparts. Preferably therefore, the polymeric particles of the present invention comprise more than one (e.g. 2 or 3) coatings. Coatings, which may be the same but are preferably different, may be formed with at least one transition metal compound as described herein. Alternatively only one of the multiple coatings, preferably the outermost coating, may be formed with at least one transition metal compound. In this latter case, other coatings present may be formed from any conventional coating material, e.g. using epoxide based coatings, polyurethane coatings or coatings made from silanes which contain e.g. the alkoxy group.

The polymer particles of the invention are preferably magnetic. By magnetic is meant herein that the polymeric particles are capable of being attracted by a magnetic field. The polymeric particles of the present invention preferably comprise paramagnetic, non superparamagnetic or superparamagnetic crystals. Paramagnetic particles will exhibit slight magnetic remanent properties. Non-superparamagnetic crystals are remanent in the sense that, upon exposure to a magnetic field, the material must have residual magnetisation in the absence of a magnetic field. The superparamagnetic polymeric particles are magnetically displaceable but are not permanently magnetizable. This means that after exposure to a magnet the particles may still be suspended or dispersed in solution without aggregation or clumping. The magnetic crystals may be of any material capable of being deposited in magnetic crystalline form within and/or on the polymeric particles. Magnetic iron oxides, e.g. magnetite or maghemite are preferred; however the crystals may be of mixed metal oxides or other magnetic material if desired. The superparamagnetic crystals are typically 5-15 nm in diameter, e.g. about 7 nm while the non-superparamagnetic (thermally blocked) iron oxide crystals are typically somewhat larger.

The magnetic polymeric particles of the present invention are preferably porous as this allows large quantities of magnetic crystals to be deposited therein. The total quantity of crystalline magnetic material present is generally more than 1%, preferably more than 3%, desirably more than or equal to 5% (by weight), e.g. more than 10%. The total quantity of crystalline magnetic material present may be up to 50% wt, preferably up to 40% wt. The percentage is calculated on a Fe (or equivalent metal in the case of magnetic materials other than iron oxides) weight basis based upon the overall dry weight of the coated particles.

Polymeric particles according to the present invention are microparticles and will have sizes (i.e. diameters) that are generally in the range 0.2 to 100 $\mu$m, e.g. 0.2 to 10 $\mu$m, preferably 0.5 to 5 $\mu$m, especially 0.8 to 1.2 $\mu$m.

Before any type of coating is put on the particles of the invention (either a metal oxide coating or other coating such as one based on an epoxide), the surface functionalised polymeric particles of use in the present invention typically have a surface area of at least 15 $m^2$/g (measured by the BET nitrogen absorption method), and more preferably at least 30 $m^2$/g, e.g. up to 700 $m^2$/g, when corrected to a mean particle diameter of 2.7 $\mu$m (i.e. multiply surface area by 2.7/MD, where MD is the mean diameter in micrometers).

The polymer particles of the invention are monodisperse. Typically the polymeric particles are spherical and monodisperse before they are coated and especially preferably remain spherical and monodisperse once they have been coated. Such particles show less variation from batch to batch than non-monodisperse particles and advantageously improve the reproducibility and reliability of data resulting from their use. Such repeatability is particularly important in particle based assays and enables, for example, use of the polymeric particles of the invention in diagnostic techniques.

By monodisperse it is meant that for a plurality of particles (e.g. at least 100, more preferably at least 1000, especially substantially all) the particles have a coefficient of variation (CV) of less than 20%, for example less than 15%, preferably less than 12%, more preferably less than 11%, still more preferably less than 10% and most preferably no more than 8%, e.g. 2 to 5%. CV is determined in percentage as

$$CV = (100 \text{ x standard deviation})/\text{mean}$$

where mean is the mean particle diameter and standard deviation is the standard deviation in particle size. CV is preferably calculated on the main mode, i.e. by fitting a monomodal distribution curve to the detected particle size distribution. Thus some particles below or above mode size may be discounted in the calculation which may for example be based on about 90% of total particle number (of detectable particles that is). Such a determination of CV is performable on a Coulter LS 130 particle size analyser.

Of particular utility in the present invention are the magnetic polymeric particles sold by Invitrogen Dynal AS (Oslo, Norway) under the trademark DYNABEADS.

Polymeric particles disclosed in WO 99/19375 (Dyno Industrier ASA) and WO 00/61648 (Dyno Specialty Polymers AS) made in part from amino styrene as disclosed in WO 0 1/70825 are particularly preferred. Functionalisation of the polymeric particles may take place after polymerisation by, for example, nitration and subsequent reduction of the thus-formed nitro groups to pendant amine groups; or direct amination, for example by treatment with amino ethanol. Polymeric particles prepared by the well-known Ugelstad two-step swelling process and the improvements thereto disclosed in WO 00/61647 (Dyno Specialty Polymers AS) may also be used. Also of use here are polymeric particles prepared by the processes disclosed in WO99/19375 and WO00/61648. Porous polymeric particles produced according to the processes described in these publications may have magnetic particles deposited in their pores by standard techniques, e.g. as described above.

Of all these processes, the use of amino styrene, particularly 4-aminostyrene, as co-monomer in the preparation of amino-bearing polymeric material is preferred. Use of this monomer or co-monomer obviates the need for post-polymerisation nitration and reduction reactions. Moreover, the more predictable nature (homogeneity) of the surface afforded by this process permits a very reliable coating to be applied.

Coating of the polymeric particles with a transition metal compound and hence an oxide generates a layer of oxide on the particle surface and optionally within the pores of the polymer particles that serves to block these pores, physically encapsulating magnetic crystals within the polymeric particles. The resulting "coated" particles therefore have reduced porosity relative to the starting material but are less prone to leaching magnetic material.

For the purposes of this application, by transition metal oxide is meant an oxide of a metal from Groups 4 or 5 of the periodic table or Al. Transition metal compounds suitable for coating the polymer particles of the invention for subsequent conversion to the oxide coating are therefore those of metals of groups 4 or 5 or Al, especially Ti, Zr and Hf or V, Nb or Ta. Highly preferably, the transition metal compound is of Nb, Ta, titanium or zirconium especially Zr or Ti.

The coating on the polymer particle is an oxide coating. Preferably this is the most stable oxide of the transition metal in question. Highly preferably the polymer particles are coated with aluminium oxide, titanium dioxide or zirconium dioxide, especially $TiO_2$ and $ZrO_2$.

Formation of the transition metal coating can be achieved by any convenient method such as chemical vapour deposition but is preferably achieved using a sol-gel technique. The particle surface prior to coating with the oxide can be un-functionalised but is preferably functionalised to carry a reactive group capable of displacing a ligand on the transition metal compound, and/or for further stabilisation by H-bonding. This enables the initial interaction between the polymer particle and the transition metal compound to take place. Conveniently, the bead surface may carry nucleophilic groups, preferably oxygen or nitrogen containing groups. The bead surface may also carry phosphonate containing groups. Thus the bead may be aminated, hydroxylated, aminohydroxylated, oximated or carry a carboxylic acid group or an aminoethanoic acid group.

Without wishing to be limited by theory, it is believed that the lability of at least one bond in the transition metal compound is critical to the success of the coating technique described herein. More specifically, it is thought upon contact with, e.g. an amine group on the surface of the polymer particles, a bond or other interaction is formed between the amine and the transition metal compound.

When such a transition metal species is contacted with water (which may well be a by-product of bond formation), the bonds on the transition metal compound may undergo a cleavage reaction to form hydroxyls (e.g. -TiOH). This compound then converts to the oxide expelling more water. Thus, whilst the initial contact of transition metal compound and bead serves to adhere the transition metal compound to the polymeric particles, the latter results in the formation of the oxide coating which is held to the bead surface through interactions such as ionic and non-ionic interactions.

The formation of the coating may therefore at least partially depend on the extent to which interaction or reaction occurs between bead and transition metal and on the number of bonds in the transition metal compound which are capable of being cleaved. Preferred transition metal compounds for use in the invention comprise 4 bonds which are capable of being cleaved upon exposure to polymer particles/water.

Preferred transition metal compounds are therefore ones which react with the polymer particle surface and can undergo ready conversion to the oxide, e.g. upon application of heat or reaction with water. Suitable transition metal compounds are therefore hydrides, halides, alcohols, oximes, alkoxides, aryloxides (e.g. phenoxides), esters (e.g. acetates, acrylates), acetoacetates, thiocarbamates, amines, alkanedionates and phosphates or mixtures of these (e.g. an alkoxy and halide carrying transition metal compound). These compounds contain at least one -Tm-H, -Tm-Hal, -TmOH, -TmNOH, -Tm-OR, -TmO(O)CR, -TmSC(S)NR, -Tm-NR, -Tm-O-CR=CR-C(O)R or -TmOP(O)OR groups respectively where R may be H, aryl, alkene, or alkyl, with up to 18C, more preferable up to 10C, especially $C_{1-6}$..

Halides and alkoxides are particularly preferred, especially alkoxides. Most especially, all ligands on the transition metal compound are alkoxy.

Preferably, the alkoxide is an alkoxide of a titanium (IV) or zirconium (IV) ion. Highly preferably the alkoxide is a C1-6 alkoxide, e.g. methoxy, ethoxy, propoxy or butoxy. Especially preferably, 4 such groups are present, e.g. tetramethoxy, tetraethoxy, tetrapropoxy or tetrabutoxy. For the purposes of clarity, is it noted that tetraalkyl zirconate, tetraalkyl orthozirconate and tetraalkoxy zirconate are the same compound. It is also possible to use a mixture of transition metal compounds to form the coating.

In a preferred process according to the present invention, polymeric particles are reacted with a transition metal alkoxide compound as hereinbefore defined in an organic solvent. Solvents of high and low water solubility may be used. The solvent is preferably one which is inert. Representative examples of suitable solvents include toluene, xylene, and ethers. A highly preferred solvent is 2-methoxyethylether. Typically 1 g of polymeric beads will be suspended in 2-150 ml of solvent, more preferably 5-15 ml.

The process to form the coated particles of the invention may be carried out in a single step or in two steps. In a single step process water may be added to a suspension or dispersion of polymeric particles and transition metal compound in an organic solvent. In the preferred two step process, the polymeric particles may first be suspended or dispersed in an organic solvent and the transition metal compound added, followed by the subsequent addition of water. In both processes an excess of transition metal compound is typically added. Preferably the reaction is carried out at a temperature in the range -10 to 200 °C, more preferably 20 to 100 °C, e.g. about room temperature. It is a surprising feature of the claimed process that the coating reaction can be effected at this low temperature and excessive heating, which could damage the polymer particle, is not required to cause formation of the coating. Typical reaction times are 10 minutes to 240 minutes, e.g. about 90 minutes.

Preferably the first step of the two step process is carried out under an inert atmosphere, e.g. under an argon atmosphere. Still more preferably water is substantially absent from the reaction. In other words, the reaction is anhydrous (e.g. it comprises < 5 % wt. water, preferably <1 % wt. water). Whilst not wishing to be bound by theory it is thought that by carrying out the first step under dry conditions only a small proportion of the transition metal compound undergoes reaction and becomes bound to the polymeric particles. Thus, the first step of the reaction serves to form a uniform "primer" layer of transition metal compound on the polymeric particles.

In the second step of the two step process water, preferably deionised water, is added. Whilst not wishing to be bound by theory, it is thought that this induces rapid hydrolysis of the transition metal compound to furnish the oxide which associates to the hydrolysed primer layer on the particle.

Typically 0.01 to 5 moles water is added per mole of transition metal compound added. At the time of addition of water, the polymeric particle suspension is typically at room temperature. Typical reaction times are 1 hour to 10 hours, preferably 1 hour to 3 hours, e.g. about 2 hours.

The processes of the invention may require the addition of acid or base, e.g. as a catalyst. However, it is preferred that the reactions occur without the addition of acids such as phosphoric acid or glacial acetic acid. It is also preferred that agitation, stirring and/or sonication be used to improve impregnation and accelerate the rate of reaction.

Following completion of the reaction the coated polymeric particles may be isolated by any conventional procedure known in the art, e.g. filtration. Generally the coated particles will be washed, preferably up to 10 times, to remove any unbound reagents such as un-reacted starting materials. Any solvent and/or mixture of solvents may be used for washing, e.g., acetone, methanol, water, isopranol or ethers. Suitably, the solvent used in the first stage is also employed as the washing solvent. The polymeric particles may then be dried, preferably under vacuum.

Thus, viewed from another aspect the invention provides a process for the preparation of polymer microparticles as hereinbefore described comprising reacting polymeric microparticles comprising polystyrene or polyacrylate with at least one transition metal alkoxide and with water. Preferably, the process involves addition of the alkoxide prior to the water.

Particles obtainable by the processes described herein form a further aspect of the invention.

## Applications

The resulting polymeric particles may be used in a wide range of applications, for example, in purification (e.g. separation, fractionation, depletion, desalting), extraction or capture (e.g. concentration) of, for example, biomolecules (e.g. peptides, proteins, nucleic acids), metal ions, organic compounds (e.g. drugs or drug derivatives), substrates and mixtures of any of the aforegoing. The polymeric particles may also be used in isolation of, for example, specific biomolecules (e.g. nucleic acids, proteins, peptides) from complex mixtures, in assays and in solid phase synthesis.

The utility of the polymeric particles of the present invention in separation and fractionation of various biomolecules facilitated through adsorption/desorption processes derives from the large binding capacity of the transition metal oxide coated particles hereinbefore described. The polymeric particles of the invention enable such separation and fractionation to be performed without a column, without sample dilution and to be automated with high throughput. The high surface area of the particles in a small volume is especially beneficial, e.g. compared to the use of plates.

In a highly preferred embodiment, the particles of the invention are used to bind nucleic acid like DNA and RNA from a sample. The isolation of DNA or RNA is an important step in many biochemical and diagnostic procedures. For example, the separation of nucleic acids from the complex mixtures in which they are often found is frequently necessary before other studies and procedures e.g. detection, cloning, sequencing, amplification, hybridisation, cDNA synthesis, studying nucleic acid structure and composition (e.g. the methylation pattern of DNA) etc. can be undertaken. The presence of large amounts of cellular or other contaminating material e.g. proteins or carbohydrates, in such complex mixtures often impedes many of the reactions and techniques used in molecular biology. In addition, DNA may contaminate RNA preparations and vice versa. Thus, methods for the isolation of nucleic acids from complex mixtures such as cells, tissues etc. are demanded, not only from the preparative point of view, but also in the many methods in use today which rely on the identification of DNA or RNA e.g. diagnosis of microbial infections, forensic science, tissue and blood typing, genotyping, detection of genetic variations etc. Isolation of nucleic acid can be achieved using the particles of the invention using any suitable nucleic acid isolation procedure.

The sample may be any material containing nucleic acid, including for example foods and allied products, clinical and environmental samples. The sample may be a biological sample, which may contain any viral or cellular material, including all prokaryotic or eukaryotic cells, viruses, bacteriophages, mycoplasmas, protoplasts and organelles. Such biological material may thus comprise all types of mammalian and non-mammalian animal cells, plant cells, algae including blue-green algae, fungi, bacteria, protozoa etc. Representative samples thus include whole blood and blood-derived products such as plasma, serum and buffy coat, urine, faeces, cerebrospinal fluid or any other body fluids, tissues, cell cultures, cell suspensions etc.

A range of methods are known for the isolation of nucleic acids, but generally speaking, these rely on a complex series of extraction and washing steps and are time consuming and laborious to perform.

US-A-5,234,809, for example, describes a method where nucleic acids are bound to a solid phase in the form of silica particles, in the presence of a chaotropic agent such as a guanidinium salt, and thereby separated from the remainder of the sample. WO 91/12079 describes a method whereby nucleic acid is trapped on the surface of a solid phase by precipitation. Generally speaking, alcohols and salts are used as precipitants.

US 5,705,628 and US 5,898,071 describe methods of isolating nucleic acid fragments using a combination of large molecular weight polyalkylene glycols (e.g. polyethylene glycols) at concentrations of from 7 to 13% with salt in the range of 0.5 to 5M to achieve binding to functional groups on a solid support which acts as a bioaffinity absorbent for DNA.

The use of the particles of the invention to isolate nucleic acid has been found to be particularly preferable. Viewed from another aspect therefore the invention provides a method for isolating nucleic acid from a sample containing nucleic acid comprising contacting said sample with the particles of the invention. More specifically, the invention provides a method for the isolation of nucleic acid from a sample comprising:

(I) providing a sample that contains one or more nucleic acids;
(II) mixing the sample with transition metal oxide coated microparticles as hereinbefore described;
(III) incubating the sample and the particles;
(IV) collecting the particles, optionally in a magnetic field and removing the supernatant; and
(V) optionally eluting one or more isolated nucleic acids.

Washing steps can be included in this process as is well known in the art, e.g. after step (IV).

In this regard, it has also been found that that nucleic acids adsorb to transition metal surfaces readily in the presence of chaotropic buffers. In a particularly preferred embodiment therefore the polymeric particles of the present invention can be used for isolation of nucleic acids, e.g. from biological specimens, by utilising this affect, i.e. in the presence of a chaotropic buffer. Adsorption of nucleic acids by the transition metal surface occurs as a result of a chaotropic buffer breaking down the hydrogen-bonded network of water surrounding the nucleic acid, thereby destabilising the structure of macromolecule in solution. Hence, by subsequently restructuring the hydrogen bonding of the surrounding environment through different washing steps, the bound nucleic acid can then be eluted from the surface, e.g. by adding a desired buffer.

Moreover, the inventors have surprisingly found that the isolation of nucleic acid using metal oxide-coated particles is highly dependent on the combination of particle surface and the buffer composition used.

Binding of nucleic acids to the polymeric particles of the invention is facilitated by the correct combination/concentration of buffer, especially chaotropic buffer and optional addition of other agents such as salts. Manipulation of temperature and pH may also assist isolation. In some cases, discrimination between different types of nucleic acids (E.g. mRNA, rRNA, tRNA, dsDNA etc.) can be obtained.

Of particular importance is the buffer employed. Suitable chaotropic buffers include guanidine salts such as the thiocyanate or chloride, urea, perchlorate salts, or iodides. Other buffers of interest include TRIS HCl, Bicine, Tricine, and phosphate buffers and mixtures of chaotropic and non chaotropic buffers may be employed. Buffers may be combined with washing buffers such as alcohols (e.g. isopropanol) and/or non-ionic surfactants such as Triton, typically of low ionic strength as is well known in the art.

The isolation may also utilise standard washing and elution buffers, e.g. those described in the examples which follow.

Hence viewed from a further aspect the invention provides a kit for the isolation of nucleic acids comprising polymeric microparticles as hereinbefore defined and a chaotropic buffer. Other optional components of the kit include washing solutions and/or elution buffers.

Incubation conditions are those typically used in the art, e.g. temperatures of from 10 to 50°C, e.g. room temperature. Incubation may be carried out for any convenient period of time e.g. 1 minute to 1 week, preferably 1 to 5 hours.

The sample can be any sample containing phosphoproteins. The preparation of such samples is well known in the art. A sample can be a cell fraction that has been lysed and preferably subjected to at least one separation procedure, e.g. extraction with one of more solutions comprising at least one salt, detergent or surfactant, an acid or base, centrifugation, solubilisation, precipitation, affinity capture, dielectrophoresis, or chromatography.

In another highly preferred embodiment, the particles of the invention are used to isolate phosphoproteins (which term shall include phosphopeptides). The microparticles of the present invention can be used for, inter alia, identification of non-protein modification or post-translational modification of cell proteins, in particular, phosphorylation. Phosphorylation and dephosphorylation regulate many cellular events, such as cell cycle control, cell differentiation, transformation, apoptosis, signal transduction etc and the microparticles of the invention provide a support for studying phosphorylation cascades by identifying proteins whose phosphorylation state can be altered by chemical, biological or even physical pertubations, such as for example, drug treatment, toxic insults, physical exposures (such as radiation or UV treatment) or stimulation with growth or differentiation factors etc.

There are various existing methods for phosphoprotein detection and isolation. Pro-Q Diamond technology (Molecular probes Inc., OR, USA) utilises a fluorophore that recognises phosphate groups on proteins without the use of antibodies or radioactivity and therefore allows simple detection of even very small amounts of phosphoproteins. Capture of phosphopeptides can be achieved by loading the fluorophore onto a magnetic bead.

Phosphoproteins can also be isolated using immobilised metal ion chromatography (IMAC) technology where columns filled with resins carrying, for example, carboxmethylated aspartate chelators with immobilised metals such as $Fe^{3+}$ are used to target phosphoproteins. Immobilized Metal Affinity Column (IMAC) chromatography is used to select out phosphopeptides from a mixture of modified and unmodified peptides in the target protein sample. A standard protocol requires the use of ferric chloride to bind to the metal chelating packing material of the IMAC column. Phosphorylated peptides will bind to the immobilized iron atoms when loaded onto the column and can be eluted under acidic conditions

Since acidic amino acid residues will also bind to the $Fe^{3+}$ on the IMAC column, non-specific binding to the column can be a hindrance. To prevent this, the sample is often treated with 2M methanolic hydrochloride prior to loading onto the IMAC column. This process converts all acidic amino acid residues, as well as the C-terminus of the peptide, from carboxylic acid groups to methyl esters. The phosphate groups remain unmodified, allowing for selective binding onto the IMAC column.

Other biochemists have used titanium dioxide particles in columns to bind phosphoproteins in combination with 2,5-dihydroxybenzoic acid to prevent non-phosphoprotein binding.

The microparticles of the present invention offer a further route to phosphoprotein isolation. Thus, viewed from a further aspect the invention provides the use of the microparticles of the invention in phosphoprotein isolation. Viewed from another aspect therefore the invention provides a method for isolating phosphoproteins from a sample containing phosphoproteins comprising contacting said sample with the particles of the invention.

Viewed from another aspect therefore, the present invention provides a method for the isolation of phosphoproteins from a sample comprising:

(I) providing a sample that contains one or more phosphoproteins;
(II) mixing the sample with transition metal oxide coated microparticles as hereinbefore described;
(III) incubating the sample and the particles;
(IV) collecting the particles, optionally in a magnetic field and removing the supernatant; and
(V) optionally eluting one or more isolated phosphoproteins.

Washing steps can be included in this process as is well known in the art, e.g. after step (IV).

The sample can be any sample containing phosphoproteins. The preparation of such samples is well known in the art. A sample can be a cell fraction that has been lysed and preferably subjected to at least one separation procedure, e.g. extraction with one of more solutions comprising at least one salt, detergent or surfactant, an acid or base, centrifugation, solubilisation, precipitation, affinity capture, dielectrophoresis, or chromatography. In some embodiments, phosphoproteins are first enriched by affinity capture using a specific binding reagent that binds one or more of phosphoserine, phosphothreonine or phosphotyrosine. Proteins also can be separated from the cell fraction by methods such as electrophoresis or chromatography. In some preferred embodiment, proteins are digested with one or more proteases to provide a sample. Preferably, the sample comprises protease digested proteins.

For example, phosphopeptides may be isolated from a complex mixture of more general peptide sequences by selective enrichment on the derivatized surface of such magnetic beads.

Incubation conditions are those typically used in the art, e.g. temperatures of from 10 to 50°C, e.g. room temperature. Incubation may be carried out for any convenient period of time e.g. 1 minute to 1 week, preferably 1 to 5 hours.

Binding of phosphoproteins to the polymeric particles of the invention is facilitated by the correct combination/concentration of buffer(s), especially chaotropic buffer and optional addition of other agents such as salts during the mixing and incubation stages. Manipulation of temperature and pH may also assist isolation.

In this regard, using conventional $TiO_2$ particles, it is normal to employ 2,5-dihydroxybenzoic acid (DHB) in the loading and/or washing buffer in relatively high concentration to ensure phosphoprotein selectivity is high. In the absence of DHB, selectivity is poor and many contaminating peptides remain.

The particles of the invention however do not require the presence of DHB in the loading or washing buffer as selectivity in its absence is excellent. Thus, in a further embodiment, Step (II) above is carried out in the absence of DHB.

The benefits of avoiding DHB are numerous as this material tends to crystallise in the highly aqueous buffer. The crystals can then clog reverse phase resins leading to back pressure and loss of sample. The use of the particles of the invention in the enrichment of phosphoproteins is therefore highly preferred.

Of particular importance to the isolation process is the buffer employed. Chaotropic buffers or detergents are useful buffers, especially binding buffers. Suitable chaotropic buffers include guanidine salts such as the thiocyanate or chloride, urea, perchlorate salts, or iodides. Other buffers of interest include Tris HCl, Bicine, Tricine, and phosphate buffers and mixtures of chaotropic and non chaotropic buffers may be employed. Buffers may be combined with washing buffers such as alcohols (e.g. isopropanol) and/or non-ionic surfactants such as Triton, typically of low ionic strength as is well known in the art.

Detergents may also be used, e.g. those described in WO96/18731. Preferred detergents include ionic, including anionic and cationic, non-ionic or zwitterionic detergents. The term "ionic detergent" as used herein includes any detergent which is partly or wholly in ionic form when dissolved in water. Anionic detergents have been shown to work particularly well and are preferred. Suitable anionic detergents include for example sodium dodecyl sulphate (SDS) or other alkali metal alkylsulphate salts or similar detergents, sarkosyl, or combinations thereof.

Combinations of detergents and chaotropic buffers may also be employed.

The isolation may also utilise standard washing buffers, e.g. those described in the examples which follow.

The inventors have surprisingly found a number of buffers which have proved particularly useful in the isolation of phosphoproteins using the microparticles of the invention. Whilst these buffers are of great utility in the presently claimed method, it will be appreciated that they are also of use in the isolation of phosphoproteins in general using a wide variety of different techniques and particles. The buffers may also have utility in the isolation of other biomolecules. These buffers therefore form a further aspect of the invention.

For binding phosphoproteins to the microparticles of the invention and for and washing the particles, the inventors have found that a 50 mM sodium acetate, pH4, 20% ethanol buffer is ideal. Such a buffer can be prepared by dissolving 1.66 g anhydrous sodium acetate in 50 ml deionised $H_2O$. After filtering with a 0.45 or 0.22 mm filter, 5.27 ml of glacial acetic acid can be added and the total volume brought up to 1.75 L with $dH_2O$. The pH of this material is 4,0 $\pm$ 0.1. 450 ml ethanol can then be added and the final volume brought up to 2.25 L by adding deionised water. All reagents used should be analytical grade

Buffers comprising water, ethanol and sodium acetate and having a pH of between 3.9 and 4.1 are thus ideal. Preferably, the ethanol forms between 10 and 30 wt% of the buffer. To achieve the desired pH the buffer may be 45 to 55 mM with respect to the sodium acetate.

In a highly preferred embodiment therefore the incubation of the phosphoprotein and microparticles of the invention takes place in the presence of this buffer.

Once separated, phosphoproteins can be eluted using conventional techniques. Elution can be in a solution which comprises one or more of piperadine, imidazole, o-phosphate, or solution which is basic, (e.g. pH 8 to 11). Preferred elution buffers include ammonium carbonate, ammonium hydroxide, diammonium citrate, ammonium acetate, ammonium dihydrogen phosphate, or ammonium bicarbonate.

A preferred elution buffer is based on phenyl phosphate and ammonium hydroxide. This is preferably a 50 mM phenyl phosphate in 0.3 N $NH_4OH$. Such a buffer can be prepared by the addition of 5 ml ammonium hydroxide (14.8 M) to 500 ml deionised water to make a 0.3 N $NH_4OH$ solution. 6.35 g of phenyl phosphate can then be added. All reagents used should be analytical grade

An elution buffer comprising phenyl phosphate (e.g. the sodium salt thereof) and ammonium hydroxide is thus ideal for use in the invention and this forms a further aspect of the invention..

Preferably, the ammonium hydroxide used to manufacture the buffer is 0.1 to 1 N. The phenyl phosphate used can be supplied as a 10 to 100 mM solution, e.g. 40 to 60 mM solution, such as 50 mM.

The inventors have surprisingly found that elution buffers optimized to work with the coated particles of the invention should contain phenylphosphate (e.g. 50 mM sodium phenyl phosphate) in ammonium hydroxide (e.g. 0.3 N ammonium hydroxide) to help compete the phosphorylated residue from the tight bind surface.

In a highly preferred embodiment therefore elution of the phosphoproteins in step (V) above is carried out using this buffer.

Once eluted the phosphoproteins can be analysed using conventional means such as mass spectrometry to identify particular proteins. The process described above is of particular utility when used in combination with samples where SILAC (stable isotopic labelling with amino acids in cell culture) is employed or where iTRAQ (isotope tagging reagents for relative absolute quantification) is employed.

The invention will now be described further by reference to the following examples and figures. These are not intended to be limitative but merely exemplary of the invention.

**Brief description of the Figures**

Figure 1 shows the isolation of dsDNA from serum (5 μg input) using:

> Lane 1-3: Magnetic beads with titanium
> Lane 4-6: Magnetic beads with zirconium
> Lane 7: 5 μg HindIII cut λ dsDNA ladder

Figure 2 shows the Isolation of RNA from serum (5 μg input) using:

> Lane 1-3: Magnetic beads with titanium
> Lane 4-6: Magnetic beads with zirconium
> Lane 7: 5 μg 0,24-9,5 Kb RNA ladder

Figure 3 shows isolation of nucleic acids from serum (5 µg input) using magnetic beads with zirconium in which:

Lane 1-3: RNA
Lane 4: 5 µg 0,24-9,5 Kb RNA ladder
Lane 5: Empty
Lane 6-8: dsDNA
Lane 9: 5 µg HindIII cut λ dsDNA ladder

Figure 4 shows isolation of nucleic acids from serum (5 µg input) using magnetic beads with zirconium:

Lane 1 and 2: dsDNA
Lane 3: 5 µg HindIII cut λ dsDNA ladder

Figure 5 shows isolation of dsDNA from serum using magnetic beads with titanium

Lane 1-2: Isolated dsDNA from 2.5 µg input
Lane 3-4: Isolated dsDNA from 5 µg input
Lane 5-6: Isolated dsDNA from 15 µg input
Lane 7-8: Isolated dsDNA from 20 µg input
Lane 9: 5 µg HindIII cut λ dsDNA ladder

Figure 6 shows isolation of genomic DNA from blood using magnetic beads with zirconium and titanium.

Lane 1-2: Isolated gDNA with zirconium Ex 1
Lane 3-4: Isolated gDNA with zirconium Ex 2
Lane 5-6: Isolated gDNA with titanium Ex 9
Lane 7-8: Isolated gDNA with titanium Ex 10
Lane 9-10: Isolated gDNA with Dynabeads® DNA DIRECT™ kit (Invitrogen Dynal AS, Oslo, Norway).

Figure 7 shows isolation of genomic DNA from blood using magnetic beads with zirconium and titanium:

Lane 1-2: Isolated gDNA with zirconium Ex 1
Lane 3-4: Isolated gDNA with zirconium Ex 2
(the reason for apparently low yield in lane 4 was the fact that gDNA was not eluted from the beads)
Lane 5-6: Isolated gDNA with titanium Ex 9
Lane 7-8: Isolated gDNA with titanium Ex 10
Lane 9-10: Isolated gDNA with Dynabeads® DNA DIRECT™ kit (Invitrogen Dynal AS, Oslo, Norway).

Figure 8 shows isolation of genomic DNA from blood using magnetic beads with titanium:

Lane 1-2: Isolated gDNA with titanium Ex 11
Lane 3-4: Isolated gDNA with titanium Ex 16

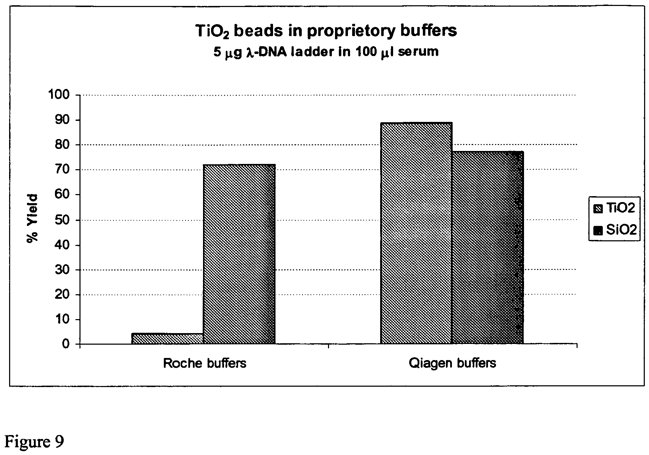

Figure 9 shows the % of DNA isolation using different proprietary buffers.

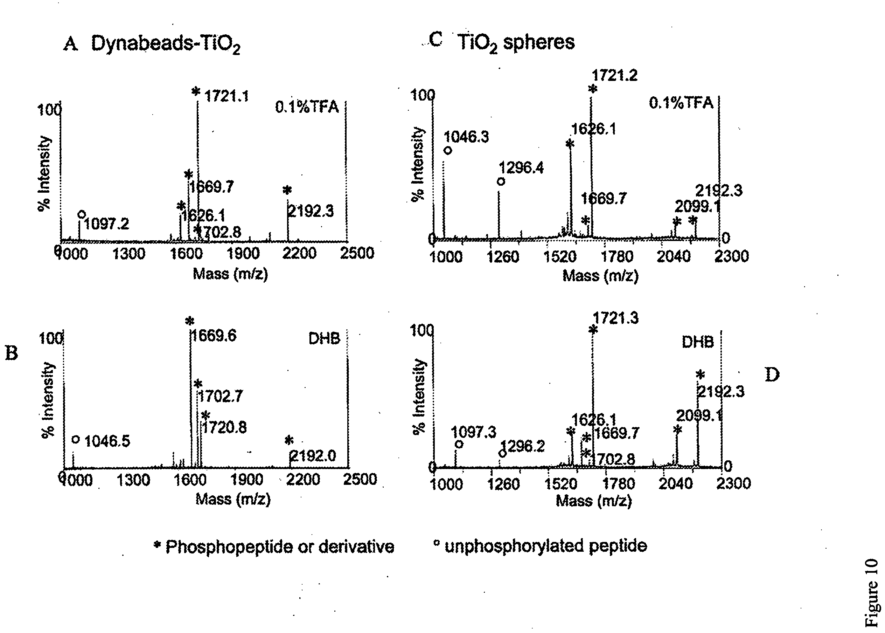

Figures 10A-D show mass spectra obtained after phosphoprotein isolation using the beads of the invention in comparison to titanium dioxide beads.

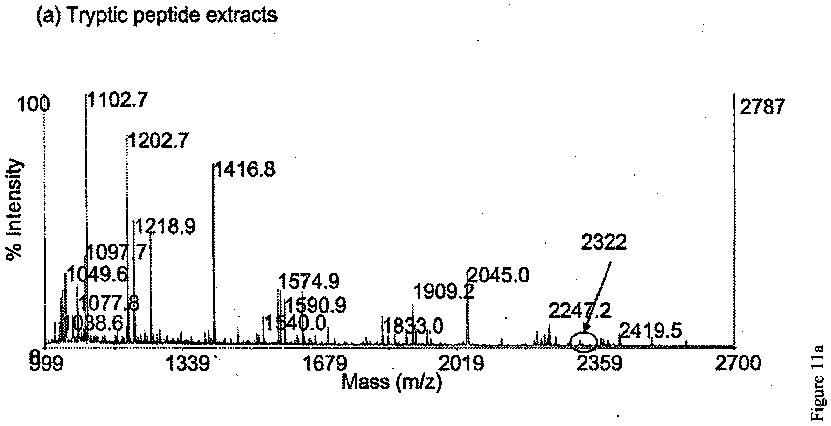

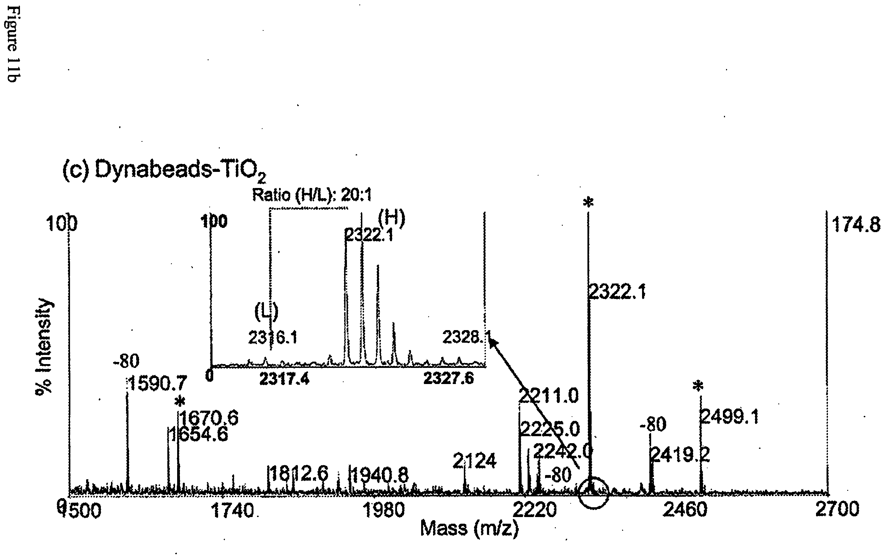

Figures 11A and B show the mass spectrum of an unknown phosphoprotein obtained by tryptic digested before and after incubation with the particles of the invention.

## EXAMPLES

### General procedures:

Starting material beads (called Beads in the examples which follow) are porous 1 µm monosized crosslinked polystyrene/divinylbenzene beads with iron oxide crystals in the pores. These magnetic polymeric particles which contain 300 - 500 mg iron/g dry solids (DS), were also coated with epoxide in a procedure described in WO 05/015216.

Each separation between the washing steps was done on magnet, and the suspension was shaken for approximately 5 minutes between each washing step.

**Zirconium Coated Particles**

**Example 1**

1.5 g of Beads were washed with bis(2-methoxyethyl ether) (washed 5 times, 10-30 mL liquid each time). The final suspension was adjusted to weigh 11.63 g.

13.71 g of tetrabutyl zirconate (80% in butanol) was added, and the suspension was stirred at 250 rpm at room temperature. After 90 min of stirring, 330 $\mu$L water was added. The suspension was stirred for another 18 hours.

After 18 hours, the particles were washed with bis(2-methoxyethyl ether) (5 times, 30-50 mL liquid each time), then washed with isopropanol (5 times, 30-50 mL liquid each time). The zirconium coating was observable by FT-IR (diffuse reflectance).

**Example 2**

2 g of Beads were washed with bis(2-methoxyethyl ether) (washed 5 times, 25-40 mL liquid each time). The final suspension was adjusted to weigh 15.95 g.

17.84 g of tetrapropyl zirconate (70% in propanol) was added, and the suspension was stirred at 250 rpm at room temperature. After 90 min of stirring, 450 $\mu$L water was added. The suspension was stirred for another 18 hours.

After 18 hours, the particles were washed with bis(2-methoxyethyl ether) (6 times, 30-50 mL liquid each time), then washed with isopropanol (7 times, 30-50 mL liquid each time), then with water (4 times, 30-50 mL liquid each time), and finally with isopropanol 4 times (30-50 mL liquid each time). The zirconium coating was observable by FT-IR (diffuse reflectance).

**Example 3**

2 g of Beads were washed with bis(2-methoxyethyl ether) (washed 5 times, 25-40 mL liquid each time). The final suspension was adjusted to weigh 15.95 g.

17.84 g of tetrapropyl zirconate (70% in propanol) and 450 $\mu$L water was added. The suspension was stirred for 18 hours at 250 rpm at room temperature.

After 18 hours, the particles were washed with bis(2-methoxyethyl ether) (5 times, 30-50 mL liquid each time), then washed with water (5 times, 30-50 mL liquid each time). The zirconium coating was observable by FT-IR (diffuse reflectance).

**Example 4**

2 g of Beads were washed with ethanol (washed 5 times, 25-40 mL liquid each time). The final suspension was adjusted to weigh 15.95 g.

17.84 g of tetrapropyl zirconate (70% in propanol) was added, and the suspension was stirred at 250 rpm at room temperature. After 90 min of stirring, 450 $\mu$L water was added. The suspension was stirred for another 18 hours.

After 18 hours, the particles were washed with ethanol 5 times (30-50 mL liquid each time), then washed with water 5 times (30-50 mL liquid each time). The zirconium coating was observable by FT-IR (diffuse reflectance).

**Example 5**

2 g of Beads were washed to bis(2-methoxyethyl ether) (washed 5 times, 25-40 mL liquid each time). The final suspension was adjusted to weigh 15.95 g.

17.84 g of tetrapropyl zirconate (70% in propanol) was added, and the suspension was stirred at 250 rpm and heated to 130°C. After 90 min of stirring at 130°C, 450 $\mu$L water was added. The suspension was stirred for another 18 hours at 130° C.

After 18 hours, the particles were washed with bis(2-methoxyethyl ether) ether 5 times (30-50 mL liquid each time), then washed with water 5 times (30-50 mL liquid each time). The zirconium coating was observable by FT-IR (diffuse reflectance).

### Example 6

1 g of Beads, epoxide coated and functionalized with carboxylic acid, were washed to bis(2-methoxyethyl ether) (washed 5 times, 25-40 mL liquid each time). The final suspension was adjusted to weigh 15.95 g.

8.92 g of tetrapropyl zirconate (70% in propanol) was added, and the suspension was stirred at 250 rpm at room temperature. After 60 min of stirring, 220 μL water was added. The suspension was stirred for another 18 hours at room temperature.

After 18 hours, the particles were washed with bis(2-methoxyethyl ether) 5 times (30-50 mL liquid each time), then washed with water 25 times (30-50 mL liquid each time). The zirconium coating was observable by FT-IR (diffuse reflectance).

### Example 7

2.5 g of Dynabeads® RPC Protein (Invitrogen Dynal AS, Oslo, Norway) were washed to bis(2-methoxyethyl ether) (washed 5 times, 30 mL liquid each time). The dry substance of particles was adjusted to 11.7 wt%. Tetrapropyl zirconate (70% in 1-propanol) (22.3 g) followed by bis(2-methoxyethyl ether) (5.0 g), was added. After stirring the mixture for one hour at 250 rpm at ambient temperature, water (0.56 g) was added. The mixture was stirred further at ambient temperature for 18 hours. The particles were washed 10 times with 50ml bis(2-methoxyethyl ether) and 20 times with 50 ml water. The zirconium coating was observable by FT-IR (diffuse reflectance).

### Example 8

4.0 g of Beads, epoxide coated, were washed to bis(2-methoxyethyl ether) (washed 5 times, 50 mL liquid each time). The dry substance of particles was adjusted to 11.7 wt%. Tetrapropyl zirconate (70% in 1-propanol) (35.7 g) followed by bis(2-methoxyethyl ether), (8.0 g) was added. After stirring the mixture for one hour at 250 rpm at ambient temperature, water (0.89 g) was added. The mixture was stirred further at ambient temperature for 18 hours. The particles were washed 10 times with 80ml bis(2-methoxyethyl ether) and 20 times with 80 ml water. The zirconium coating was observable by FT-IR (diffuse reflectance).

### Titanium Coatings

### Example 9

2 g of Beads were washed to bis(2-methoxyethyl ether) (washed 5 times, 25-40 mL liquid each time). The final suspension was adjusted to weigh 14.29 g.

8.70 g of tetraethyl orthotitanate was added, and the suspension was stirred at 250 rpm at room temperature. After 90 min of stirring, 210 μL water was added. The suspension was stirred for another 2 hours.

After 2 hours, the particles were washed with bis(2-methoxyethyl ether)10 times (30-50 mL liquid each time), then washed with acetone 4 times (30-50 mL liquid each time), and finally washed with water 4 times (30-50 mL liquid each time). The titanium coating was observable by FT-IR (diffuse reflectance).

### Example 10

1.8 g of Beads were washed to bis(2-methoxyethyl ether) (washed 5 times, 35-60 mL liquid each time). The final suspension was adjusted to weigh 37.64 g.

13.05 g of tetraethyl orthotitanate was added, and the suspension was stirred at 250 rpm at room temperature. After 90 min of stirring, 670 μL water was added. The suspension was stirred for another 2 hours.

After 2 hours, the particles were washed with bis(2-methoxyethyl ether) 9 times (approx. 100 mL liquid each time), then washed with isopropanol 4 times (approx. 100 mL liquid each time). The titanium coating was observable by FT-IR (diffuse reflectance).

### Example 11

1.8 g of Beads were washed to bis(2-methoxyethyl ether) (washed 5 times, 35-60 mL liquid each time). The final suspension was adjusted to weigh 37.64 g.

19.46 g of tetrabutyl orthotitanate was added, and the suspension was stirred at 250 rpm at room temperature. After 90 min of stirring, 670 μL water was added. The suspension was stirred for another 2 hours.

After 2 hours, the particles were washed with bis(2-methoxyethyl ether) 9 times (approx. 100 mL liquid each time), then washed with isopropanol 4 times (approx. 100 mL liquid each time). The titanium coating was observable by FT-IR (diffuse reflectance).

## Example 12

2.0 g of Beads were washed to bis(2-methoxyethyl ether) (washed 5 times, 25 mL liquid each time). The dry substance of particles was adjusted to 9.5 wt% and tetrabutyl orthotitanate (13.0 g) was added. After stirring the mixture for one hour at ambient temperature, water (0.45 g) was added. The mixture was stirred further at ambient temperature for 18 hours. The particles were washed 6 times with bis(2-methoxyethyl ether) and 5 times with water. The titanium coating was observable by FT-IR (diffuse reflectance).

## Example 13

1.5 g of Beads were washed to bis(2-methoxyethyl ether) (washed 5 times, 20-40 mL liquid each time). The final suspension was adjusted to weigh 18.82 g.
6.52 g of tetrabutyl orthotitanate was added, and the suspension was stirred at 250 rpm at room temperature. After 90 min of stirring, 330 $\mu$L HNO$_3$-solution (pH=1) was added. The suspension was stirred for another 18 hours.
After 18 hours, the particles were washed with bis(2-methoxyethyl ether) 9 times (approx. 50 mL liquid each time), then washed with isopropanol 4 times (approx. 1050 mL liquid each time). The titanium coating was observable by FT-IR (diffuse reflectance).

## Example 14

1.5 g of Beads were washed to bis(2-methoxyethyl ether) (washed 5 times, 20-40 mL liquid each time). The final suspension was adjusted to weigh 18.82 g.
6.52 g of tetrabutyl orthotitanate was added, and the suspension was stirred at 250 rpm at room temperature. After 90 min of stirring, 330 $\mu$L NH$_4$OH-solution (pH=11) was added. The suspension was stirred for another 18 hours.
After 18 hours, the particles were washed with 2 bis(2-methoxyethyl ether) 9 times (approx. 50 mL liquid each time), then washed with isopropanol 4 times (approx. 50 mL liquid each time). The titanium coating was observable by FT-IR (diffuse reflectance).

## Example 15

1 g of Beads, epoxide coated and functionalized with carboxyl acid, were washed to bis(2-methoxyethyl ether) (washed 5 times, 20-40 mL liquid each time). The final suspension was adjusted to weigh 10.55 gram.
6.52 g of tetrabutyl orthotitanate was added, and the suspension was stirred at 250 rpm at room temperature. After 60 min of stirring, 220 $\mu$L water was added. The suspension was stirred for another 18 hours.
After 18 hours, the particles were washed with bis(2-methoxyethyl ether) 5 times (approx. 50 mL liquid each time), then washed with water 20 times (approx. 50 mL liquid each time). The titanium coating was observable by FT-IR (diffuse reflectance).

## Example 16

4 g of epoxide coated Beads containing approx. 335 mg Fe /g DS, were washed to bis(2-methoxyethyl ether) (washed 5 times, 50-80 mL liquid each time). The final suspension was adjusted to weigh 42.22 g.
25.95 g of tetrabutyl orthotitanate was added, and the suspension was stirred at 250 rpm at room temperature. After 60 min of stirring, 890 $\mu$L water was added. The suspension was stirred for another 18 hours.
After 18 hours, the particles were washed with bis(2-methoxyethyl ether) 5 times (approx. 80 mL liquid each time), then washed with water 5 times (approx. 80 mL liquid each time). The titanium coating was observable by FT-IR (diffuse reflectance).

## Example 17

2.0 g of epoxy coated Beads, containing approx. 435 mg Fe/g DS, were washed to bis(2-methoxyethyl ether) (washed 5 times, 25 mL liquid each time). The dry substance of particles was adjusted to 9.5 wt% and tetrabutyl orthotitanate (13.0 g) was added. After stirring the mixture for one hour at ambient temperature, water (0.45 g) was added. The mixture was stirred further at ambient temperature for 18 hours. The particles were washed 6 times with bis(2-methoxyethyl ether) and 7 times with water. The titanium coating was observable by FT-IR (diffuse reflectance).

**Example 18**

2.0 g of Dynabeads® RPC 18 (Invitrogen Dynal AS, Oslo Norway) were washed to bis(2-methoxyethyl ether) (washed 5 times, 25 mL liquid each time). The dry substance of particles was adjusted to 11.7 wt%. Tetrabutyl orthotitanate (13.0 g) followed by bis(2-methoxyethyl ether) (4.0 g) was added. After stirring the mixture for one hour at 250 rpm at ambient temperature, water (0.45 g) was added. The mixture was stirred further at ambient temperature for 18 hours. The particles were washed 6 times with 50ml bis(2-methoxyethyl ether) and 70 times with 50 ml water. The titanium coating was observable by FT-IR (diffuse reflectance).

**Example 19**

2.5 g of Dynabeads® RPC Protein (Invitrogen Dynal AS, Oslo, Norway) were washed to bis(2-methoxyethyl ether) (washed 5 times, 30 mL liquid each time). The dry substance of particles was adjusted to 11.7 wt%. Tetrabutyl orthotitanate (16.2 g) followed by bis(2-methoxyethyl ether) (5.0 g) was added. After stirring the mixture for one hour at 250 rpm at ambient temperature, water (0.56 g) was added. The mixture was stirred further at ambient temperature for 18 hours. The particles were washed 10 times with 50ml bis(2-methoxyethyl ether) and 20 times with 50 ml water. The titanium coating was observable by FT-IR (diffuse reflectance).

**Application of the Particles of the invention in bioseparation**

**Nucleic Acid isolation:**

**General Protocols and materials used:**

Source of Nucleic acid:

dsDNA HindIII λ-DNA ladder commercially from Invitrogen Corp, Carlsbad, CA, USA
RNA 0,24-9,5 Kb RNA ladder commercially from Invitrogen Corp, Carlsbad, CA, USA

Commercially available buffers and solutions used:

(*) from Ambion (TX, USA) in the MagMAX™ Viral RNA isolation kit.
(**) from Invitrogen Dynal AS (Oslo, Norway) in the DNA DIRECT™ Blood kit
(***) from Invitrogen Dynal AS (Oslo, Norway) in the DNA DIRECT™ Universal kit
(****) from Qiagen (CA, USA) in the MagAttract® Virus Mini M48 kit
(*****) from Roche Diagnostic GmbH (Mannheim, Germany) in the MagNA Pure Total Nucleic Acid kit
(******) from Invitrogen Dynal AS (Oslo, Norway) in the Dynabeads® gDNA Silane kit

The yields of RNA and dsDNA were determined by spectrophotometric analysis and ethidium bromide stained agarose gel electrophoresis.

**Example 20**

**Purification of double stranded DNA from serum using titanium dioxide (Example 10) and zirconium dioxide (Example 2) magnetic beads**

In a study demonstrating the ability of the titanium dioxide and zirconium dioxide magnetic beads to bind and elute dsDNA in an automated system, a total amount of 5 μg dsDNA was added to 2 mg beads of Example 2 or 10 in a tube containing 100 μl serum, 100 μl Proteinase K, 150 μl 100% isopropanol and 300 μl of guanidine thiocyanate containing lysis/binding solution (*). The solution was incubated at room temperature for 10 minutes with constant mixing. After collecting the beads in a magnetic field the supernatant was removed. The bead pellet was then washed with 850 μl of a guanidine thiocyanate and isopropanol containing solution (*). The supernatant was removed from the beads after the beads were collected in a magnetic field. The bead pellet was then washed twice with 450 μl of an ethanol containing solution (*). The supernatant was removed from the beads after the beads were collected in a magnetic field. The dsDNA was eluted in 100 μl elution buffer (*) by heating the bead solution to 80°C for 10 minutes with constant mixing. The beads were collected in a magnetic field and the supernatant containing the eluted dsDNA was removed from the beads and transferred to an new tube. The dsDNA yield is shown in figure 1 and table 1.

**Table 1**

| Bead | Lane | ng/$\mu$l | %yield | Average % yield |
|---|---|---|---|---|
| Titanium | 1 | 24,7 | 49 | |
| | 2 | 29,9 | 60 | 56 |
| | 3 | 28,8 | 58 | |
| Zirconium | 4 | 32,1 | 64 | |
| | 5 | 36,5 | 73 | 66 |
| | 6 | 31,1 | 62 | |
| Yield of 5 $\mu$g input dsDNA isolated from serum using magnetic beads with titanium and zirconium. | | | | |

### Example 21

**Purification of RNA from serum using titanium dioxide (Example 10) and zirconium dioxide (Example 2) magnetic beads**

In a study demonstrating the ability of the titanium dioxide and zirconium dioxide magnetic beads to bind and elute RNA in an automated system, a total amount of 5 $\mu$g RNA was added to 2 mg beads of Examples 2 and 10 in a tube containing 100 $\mu$l serum, 100 $\mu$l Proteinase K, 150 $\mu$l 100% isopropanol and 300 $\mu$l of a guanidine thiocyanate containing lysis/binding solution (*). The solution was incubated at room temperature for 10 minutes with constant mixing. After collecting the beads in a magnetic field the supernatant was removed. The bead pellet was then washed with 850 $\mu$l of a guanidine thiocyanate and isopropanol containing solution (*). The supernatant was removed from the beads after the beads were collected in a magnetic field. The bead pellet was then washed twice with 450 $\mu$l of an ethanol containing solution (*). The supernatant was removed from the beads after the beads were collected in a magnetic field. The RNA was eluted in 100 $\mu$l elution buffer (*) by heating the bead solution to 80°C for 10 minutes with constant mixing. The beads were collected in a magnetic field and the supernatant containing the eluted RNA was removed from the beads and transferred to an new tube. The RNA yield is shown in figure 2 and table 2.

**Table 2**

| Bead | Lane | ng/$\mu$l | % yield | Average % yield |
|---|---|---|---|---|
| Titanium | 1 | 17,2 | 34 | |
| | 2 | 22,6 | 45 | 40 |
| | 3 | 20,2 | 40 | |
| Zirconium | 4 | 27,7 | 55 | |
| | 5 | 30,3 | 61 | 58 |
| | 6 | 29,3 | 59 | |
| Yield of 5 $\mu$g input RNA isolated from serum using magnetic beads with titanium and zirconium. | | | | |

### Example 22

**Purification of nucleic acids from serum using zirconium dioxide magnetic beads (Example 2)**

In a study demonstrating the ability of the zirconium dioxide magnetic beads to bind and elute RNA and DNA in an automated system, a total amount of 5 $\mu$g RNA and 5 $\mu$g dsDNA was added to 2 mg beads in a tube containing 100 $\mu$l serum, 100 $\mu$l Proteinase K, 150 $\mu$l 100% isopropanol and 300 $\mu$l of a lysis/binding solution containing 4-6 M guanidine thiocyanate, 10-20% Triton X-100, 50 mM Tris-HCl, pH 6-8. The solution was incubated at room temperature for 10 minutes with constant mixing. After collecting the beads in a magnetic field the supernatant was removed. The bead pellet was then washed with 850 $\mu$l of a guanidine thiocyanate and isopropanol containing solution (*). The supernatant was removed from the beads after the beads were collected in a magnetic field. The bead pellet was then washed twice with 450 $\mu$l of an ethanol containing solution (*). The supernatant was removed from the beads after the beads were collected in a magnetic field. The RNA was eluted in 100 µl elution buffer (*) by heating the bead solution to 80°C for 10 minutes with constant mixing. The beads were collected in a magnetic field and the supernatant containing the eluted nucleic acid was removed from the beads and transferred to an new tube. The nucleic acid yield is shown in figure 3 and table 3.

**Table 3**

| Nucleic acid | Lane | ng/µl | % yield | Average % yield |
|---|---|---|---|---|
| RNA | 1 | 22,6 | 45 | |
| | 2 | 19,8 | 40 | 43 |
| | 3 | 22,8 | 46 | |
| DNA | 6 | 23,0 | 46 | |
| | 7 | 25,0 | 50 | 49 |
| | 8 | 24,8 | 50 | |
| Yield of 5 µg input nucleic acid isolated from serum using magnetic beads with zirconium. | | | | |

**Example 23**

**Purification of double stranded DNA from serum using zirconium dioxide magnetic beads (Example 1)**

In a study demonstrating the ability of the zirconium dioxide magnetic beads to bind and elute dsDNA, a total amount of 5 µg dsDNA was added to 2 mg beads in a tube containing 100 µl serum and 800 µl of a guanidine thiocyanate containing lysis/binding solution (*). The solution was vortexed carefully for 4 minutes. After collecting the beads in a magnetic field the supernatant was removed. The bead pellet was then washed twice with 400 µl of a guanidine thiocyanate and isopropanol containing solution (*). The supernatant was removed from the beads after the beads were collected in a magnetic field. The bead pellet was then washed twice with 400 µl of an ethanol containing solution (*). The supernatant was removed from the beads after the beads were collected in a magnetic field. The bead pellet was air dried for 2 minutes before the dsDNA was eluted in 100 µl elution buffer (*) by heating the bead solution to 70°C for 3 minutes. The beads were collected in a magnetic field and the supernatant containing the eluted dsDNA was removed from the beads and transferred to an new tube. The dsDNA yield is shown in figure 4 and table 4.

**Table 4**

| Lane | ng/µl | % yield | Average % yield |
|---|---|---|---|
| 1 | 41,7 | 83 | 79 |
| 2 | 36,9 | 74 | |
| Yield of 5 µg input dsDNA isolated from serum using magnetic beads with zirconium. | | | |

**Example 24**

**Purification of double stranded DNA from serum using titanium dioxide magnetic beads (Example 9)**

In a study demonstrating the ability of the titanium dioxide magnetic beads to bind and elute dsDNA, an increasing amount (2.5, 5, 15 and 20 µg) of dsDNA was added to 2 mg beads in a tube containing 100 µl serum and 800 µl of a guanidine thiocyanate containing lysis/binding solution (*). The solution was vortexed carefully for 4 minutes. After collecting the beads in a magnetic field the supernatant was removed. The bead pellet was then washed twice with 400 µl of a guanidine thiocyanate and isopropanol containing solution (*). The supernatant was removed from the beads after the beads were collected in a magnetic field. The bead pellet was then washed twice with 400 µl of an ethanol containing solution (*). The supernatant was removed from the beads after the beads were collected in a magnetic field. The bead pellet was air dried for 2 minutes before the dsDNA was eluted in 100 µl elution buffer (*) by heating the bead solution to 70°C for 3 minutes. The beads were collected in a magnetic field and the supernatant containing the eluted dsDNA was removed from the beads and transferred to an new tube. The dsDNA yield is shown in figure 5 and table 5.

**Table 5**

| Lane | μg input dsDNA | ng/μl | % yield | Average % yield |
|------|----------------|-------|---------|-----------------|
| 1 | 2,5 | 28,3 | 100 | 100 |
| 2 | | 27,1 | 100 | |
| 3 | 5 | 52,8 | 100 | 100 |
| 4 | | 48,9 | 98 | |
| 5 | 15 | 129,4 | 86 | 86 |
| 6 | | 127,8 | 85 | |
| 7 | 20 | 167,6 | 84 | 88 |
| 8 | | 181,6 | 91 | |
| Yield of increasing amounts of input dsDNA isolated from serum using magnetic beads with titanium. | | | | |

**Example 25**

**Purification of genomic DNA from blood using titanium dioxide (Example 9 and Example 10) and zirconium dioxide (Example 1 and Example 2) magnetic beads.**

In a study the ability of the titanium dioxide and zirconium dioxide magnetic beads to bind and elute genomic DNA was demonstrated. 100 μl EDTA blood was added 1 ml Red Cell Lysis buffer (**). The solution was incubated at room temperature on a roller for 5 minutes. The white blood cells were pelleted by centrifugation at 14000 rpm and the supernatant was removed. 0.5 mg beads were added together with 200 μl Lysis/Binding buffer without the beads from the kit (**). The bead suspension was not mixed further, but was left for 5 minutes at room temperature. The bead pellet was then washed carefully with 1 ml 1x Washing buffer (**) without resuspending the pellet. After collecting the beads in a magnetic field the supernatant was removed. The washing step was repeated twice. The bead pellet was added 100 μl Resuspension buffer (**) and the beads were resuspended by pipetting. The bead suspension was heated for 5 min at 65°C. The bead suspension was mixed by pipetting before collecting the beads in a magnetic field. The supernatant was transferred to a new tube. The isolated genomic DNA analysed on an ethidium bromide stained agarose gel shown in figure 6.

**Example 26**

**Purification of genomic DNA from blood using titanium dioxide (Example 9 and Example 10) and zirconium dioxide (Example 1 and example 2) magnetic beads.**

In a study the ability of the titanium dioxide and zirconium dioxide magnetic beads to bind and elute genomic DNA was demonstrated. 10 μl EDTA blood was added 0.5 mg beads together with 200 μl Lysis/Binding buffer without the beads from the kit (***). The bead suspension was not mixed further, but was left for 5 minutes at room temperature. The bead pellet was then washed carefully with 200 μl lxWashing buffer (***) without resuspending the pellet. After collecting the beads in a magnetic field the supernatant was removed. The washing step was repeated once. The bead pellet was added 20 μl Resuspension buffer (***) and the beads were resuspended by pipetting. The bead suspension was heated for 5 min at 65°C. The bead suspension was mixed by pipetting before collecting the beads in a magnetic field. The supernatant was transferred to a new tube. The isolated genomic DNA analysed on an ethidium bromide stained agarose gel shown in figure 7.

**Example 27**

**Purification of genomic DNA from blood using titanium dioxide (Example 12 and Example 17)**

50 μl Proteinase K (20 mg/ml) was added to 350μl blood, mixed and incubated for 2 min at room temperature. 350 μl of Lysis Buffer (******) was added and mixed. After incubating for 10 min at 55°C, 2.0 mg beads were added and mixed with the sample. 400 μl of isopropanol was added and mixed with beads and sample and incubate for 3 min on a roller. The beads were collected in a magnetic field and the supernatant removed. Thereafter the beads were washed twice with 1 ml Washing Buffer 1 (\*\*\*\*\*\*). The bead pellet was resuspended in 1 ml Washing Buffer 2 (\*\*\*\*\*\*), and transferred to new tubes. The beads were collected in a magnetic field and the supernatant removed followed by a final wash of the beads with 1 ml Washing Buffer 2 (\*\*\*\*\*\*).The beads were pelleted, and after removing the supernatant, air dried for 5 - 10 min.100 $\mu$l elution buffer (\*\*\*\*\*\*) was added and the bead suspension was mixed by pipetting before collecting the beads in a magnetic field. The supernatant with the eluted DNA was transferred to a new tube. The isolated genomic DNA was analysed on an ethidium bromide stained agarose gel shown in figure 8.

**Example 28**

**Purification of double stranded DNA from serum using titanium dioxide (Example 9) magnetic beads and different buffer compositions.**

In a study demonstrating that the titanium dioxide needs a particular buffer composition to bind and elute dsDNA two different commercial kits containing magnetic silica based beads were used. The protocols were performed by exchanging the magnetic beads in the kits with titanium dioxide magnetic beads from example 9. The first protocol was performed by adding a total amount of 5 $\mu$g dsDNA to 100 $\mu$l serum, 130 $\mu$l Lysis AL buffer (\*\*\*\*) and 20 $\mu$l Proteinase (\*\*\*\*). This was incubated at 56°C for 15 minutes. 2 mg beads of example 9 and 130 $\mu$l 100% isopropanol were added to the mixture and this was incubated at room temperature for 10 minutes with constant mixing. After collecting the beads in a magnetic field the supernatant was removed. The bead pellet was then washed with 400 $\mu$l AW1 (\*\*\*\*).The supernatant was removed from the beads after the beads were collected in a magnetic field. The bead pellet was then washed with 400 $\mu$l AW2 (\*\*\*\*). The supernatant was removed from the beads after the beads were collected in a magnetic field. The bead pellet was then washed with 400 $\mu$l 96% ethanol. The supernatant was removed from the beads after the beads were collected in a magnetic field. The dsDNA was eluted in 100 $\mu$l elution buffer (\*\*\*\*). The beads were collected in a magnetic field and the supernatant containing the eluted dsDNA was removed from the beads and transferred to an new tube.

The second protocol was performed by adding a total amount of 5 $\mu$g dsDNA to 2 mg beads of example 9 in a tube containing 100 $\mu$l serum, 100 $\mu$l Proteinase K (\*\*\*\*\*), 150 $\mu$l 100% isopropanol and 300 $\mu$l Lysis Binding buffer (\*\*\*\*\*). This was incubated at room temperature for 10 minutes with constant mixing. After collecting the beads in a magnetic field the supernatant was removed. The bead pellet was then washed with 850 $\mu$l Wash buffer I (\*\*\*\*\*).The supernatant was removed from the beads after the beads were collected in a magnetic field. The bead pellet was then washed with 450 $\mu$l Wash buffer II (\*\*\*\*\*).The supernatant was removed from the beads after the beads were collected in a magnetic field. The bead pellet was then washed with 450 $\mu$l Wash buffer III (\*\*\*\*\*). The supernatant was removed from the beads after the beads were collected in a magnetic field. The dsDNA was eluted in 100 $\mu$l elution buffer (\*\*\*\*\*). The beads were collected in a magnetic field and the supernatant containing the eluted dsDNA was removed from the beads and transferred to an new tube.

Results are presented in Figure 9.

**Phosphoprotein Isolation**

**Example 29**

A phosphopeptide standard mixture [Angiotensin II (DRVYIHPF, 1046.54), Angiotensin I (DRVYIHPFHL, 1296.88), Myelin basic protein fragment 104-118 (GKGRGLSLSRFSWGA, 1578.85), pTpY peptide (MAP kinase fragment 177-189, DHTGFLpTEpYVATR, 1669.67), pY peptide (Insulin receptor fragment 1142-1153 (TRDIpYETDYYRK, 1702.75), pT peptide (VPIPGRFDRRVpTVE, 1720.89), pS peptide (RII phosphopeptide fragment 81-99, DLDVPIPGRFDRRVpSVAAE, 2192.08)] was obtained from Invitrogen Corp., CA, USA).

The phosphopeptide standard mixture was isotopically labelled with iTRAQ (Invitrogen Corp., CA, USA) 114,115,116, or 117 according to manufacturer's instructions.

Aliquots of iTRAQ labelled peptides (5 $\mu$l of 2 pmol/$\mu$l stock) were acidified with 2 $\mu$l of 50% acetic acid, diluted with 36 $\mu$l binding buffer (0.1% TFA or 300 mg/ml DHB/80% acetonitrile/0.1% TFA), and then incubated with 8 $\mu$l of 50 mg/ml the beads of Example 9 for 10 min or they were passed through an Eppendorf® GELoader® tip (Eppendorf A.G., Hamburg, Germany) either prepacked with Poros® 20MC (Applied Biosystems, CA, USA) (approximately 3 mm packing length) or titanium dioxide spheres purchased from GL Sciences Inc, (Torrance, CA) to compare the relative efficiency of phosphopeptide enrichment.

After extensive washing (80% acetonitrile in 0.1 % TFA), phosphopeptides were eluted with 10 $\mu$l of elution buffer (50 mM sodium phenylphosphate in 0.3 N ammonium hydroxide.

To quantitatively compare capture efficiency, phosphopeptides isolated by the two different affinity chemistries and two different supports, the eluted material was mixed and acidified with 4 $\mu$l of 50% acetic acid, followed by desalting with a self-packed Poros® R2 tip column (Applied Biosystems, CA, USA). Phosphopeptides were eluted directly onto a MALDI plate and mixed with CHCA (alpha-cyano-4-hydroxycinnamic acid) matrix at 1:1 ratio, followed by analysis using a 4700 Proteomics Analyzer (Applied Biosystems, CA., USA).

Prior to sample analysis, the CID gas cell was purged in order to obtain stable isotopic ratios within the reporter ion. A total of 14,000 laser shots were used to average the signals of fragmenting ions. Quantification was done manually by examination of the intensity ratio of reporter ions in the MS/MS (tandem MS) spectra.

The results obtained are presented in Figures 10A to D. The peaks marked with a circle are contaminant peaks. It is clear that the presence of DHB is essential to optimize specific enrichment of phosphopeptides using titanium dioxide spheres. In contrast, the presence of DHB has little effect on the specificity of enrichment using the magnetic titanium dioxide coated "Dynabead-TiO$_2$" particles of Example 9. Moreover, the isolation performance of the particles of the invention is better than either comparative separation using titanium dioxide particles.

### Example 30

HeLa cells (ATCC) were maintained in DMEM medium (Invitrogen Corp., CA., USA) containing 10% FBS (Invitrogen Corp., CA., USA). For SILAC labelling, aliquots of HeLa cells were propagated for at least six doublings (approximately 10 days) in SILAC DMEM media containing 10% dialyzed FBS and supplemented with light L-lysine and light L-arginine (light medium) or heavy [U-$^{13}$C6] L-lysine and heavy [U-$^{13}$C$_6$,$^{15}$N$_4$] L-arginine (heavy medium). After overnight starvation with the corresponding serum-free light or heavy medium, HeLa cells labelled with heavy medium (50x 10$^6$ cells) were stimulated with 150 ng/ml EGF for 5 minutes while HeLa cells labelled with light medium (50 x 10$^6$ cells) remained untreated. Upon stimulation, heavy-labelled cells and light-labelled cells were lysed immediately in NP-40 lysis buffer containing 50 mM Tris-HCl (pH 8.0), 150 mM NaCl, 1% NP-40, 0.1% sodium deoxycholate, 1 mM Na$_3$VO4,10 mM NaF, and protease inhibitor cocktail.

Cell lysates were clarified by centrifugation at 100,000 x g for 15 minutes and the mixed cell lysates were immunoprecipitated with approximately 50 μg of either phosphotyrosine antibody (PY-Plus) conjugated to Dynabeads or 4G10 phosphotyrosine antibody conjugated to agarose beads. After mixing by gentle rotation for 2 h at 4°C, the Dynabeads were captured with a magnet, whereas the agarose beads were harvested by centrifugation. The beads were washed five times with lysis buffer and then eluted with 50 μl of 100 mM glycine (pH 2.5). The eluates were neutralized with 1 M Tris, reduced with 10 mM DTT and alkylated with 30 mM iodoacetamide in 1 x SDS sample buffer, and then separated on SDS-PAGE. Protein bands were excised from gels and subjected to in-gel tryptic digestion. Peptide extracts were dried using a Speed Vac.

The extract was incubated with 250 μg of the particles of Example 9 for 10 minutes in 24 μl of binding buffer to isolate phosphopeptides. The particles were washed five times with washing buffer and then eluted with 10 μl of elution buffer. The eluted phosphopeptides were acidified with 2 μl of 50% acetic acid and then desalted with a self-packed PorosR2 tip column, followed by analysis using MALDI-TOF-TOF.

Figure 11a shows a typical MS of an extract before incubation with the particles of the invention. Figure 11b shows the MS of the same extract after incubation. The spectrum is greatly simplified making it easy to identify captured phosphopeptides. The peak at 2322.1 was identified as the heavy 13C6-Lys-labelled phosphopeptide GSHQISLDNPDpYQQDFFPK derived from EGFR.

The results show that quantification in the level of phosphorylation at specific residues is achievable.

### Claims

1. Monodisperse polymer microparticles having a coefficient of variation (CV) of less than 20% and comprising polystyrene or polyacrylate, wherein said particles have a coating formed of at least one transition metal oxide of a group 4 or 5 metal or Al or a mixture thereof.

2. Microparticles as claimed in claim 1, which are magnetic, optionally where the magnetic material is adsorbed onto the surface of the particles prior to the coating of the at least one transition metal oxide.

3. Microparticles as claimed in claim 1 or claim 2, which are porous, optionally wherein the microparticles are magnetic and where the magnetic material is deposited in the pores of the particles prior to the coating of the at least one transition metal oxide.

4. Microparticles as claimed in any preceding claim, which are covered by an additional polymeric coating layer prior to the coating of the at least one transition metal oxide.

5. Microparticles as claimed in any preceding claim, whose diameters have a coefficient of variation of no more than 8%, optionally wherein the microparticles have diameters of 0.2 to 10 μm.

6. Microparticles as claimed in any preceding claim, wherein the transition metal oxide is titanium, tantalum, niobium or zirconium oxide.

7. Microparticles as claimed in any preceding claim being styrene divinylbenzene particles.

8. A process for the preparation of monodisperse polymer microparticles having a coefficient of variation (CV) of less than 20% as hereinbefore described comprising reacting polymeric microparticles comprising polystyrene or polyacrylate with at least one transition metal compound of a group 4 or 5 metal or Al or a mixture thereof capable of being converted into an oxide and forming the transition metal oxide coating, e.g, upon application of heat and/or water.

9. A process as claimed in claim 8, wherein prior to coating the microparticles, the starting microparticles are surface functionalised, optionally wherein the microparticles are surface functionalised to carry amino, hydroxyl, epoxide, carboxy or siloxy groups.

10. A process as claimed in claim 8 or claim 9 wherein the transition metal compound is an alkoxide.

11. A process as claimed in claim 10 wherein the alkoxide is a tetra (C1-6)zirconium or titanium alkoxide.

12. A process as claimed in any one of claims 8 to 11 carried out in at least two steps, a first step comprising contacting the microparticles with transition metal compound in an anhydrous environment and a second step comprising adding water to the product of the first step.

13. A method for the isolation of phosphoproteins from a sample containing phosphoproteins comprising contacting said sample with the monodisperse polymer microparticles of claims 1 to 7, the method optionally comprising:

    (I) providing a sample that contains one or more phosphoproteins;
    (II) mixing the sample with monodisperse polymer microparticles;
    (III) incubating the sample and the particles;
    (IV) collecting the particles, optionally in a magnetic field and removing the supernatant; and optionally
    (V) eluting one or more isolated phosphoproteins.

14. A method as claimed in claim 13 wherein step (V) is carried out in the presence of a buffer comprising phenyl phosphate and ammonium hydroxide.

15. A method as claimed in claim 13 or claim 14, wherein step (II) is carried out in the presence of a buffer comprising water, ethanol and sodium acetate and having a pH of between 3.9 and 4.1.

16. A method as claimed in any of claims 13 to 15, wherein step (II) is carried out in the absence of 2, 5-dihydroxybenzoic acid.

17. Use of the polymer microparticles as claimed in any of claims 1 to 7 in phosphoprotein isolation.

18. A method for isolating nucleic acid from a sample containing nucleic acid comprising contacting said sample with monodisperse polymer microparticles as claimed in claim 1 to 7, the method optionally comprising:

    (I) providing a sample that contains one or more nucleic acids;
    (II) mixing the sample with transition metal oxide coated microparticles as hereinbefore described;
    (III) incubating the sample and the particles;
    (IV) collecting the particles, optionally in a magnetic field and removing the supernatant; and
    (V) optionally eluting one or more isolated nucleic acids.

19. A method as claimed in claim 18 in which contact between the nucleic acid and microparticles occurs in the presence of a chaotropic buffer.

20. A kit for the isolation of nucleic acids comprising polymer microparticles as described in claims 1 to 7 and a chaotropic buffer.

**Patentansprüche**

1. Monodisperse Polymer-Mikroteilchen, die einen Variationskoeffizienten (CV) von weniger als 20 % aufweisen und Polystyrol oder Polyacrylat enthalten, wobei die Partikel eine Beschichtung haben, die aus mindestens einem Übergangsmetalloxid eines Gruppe-4- oder Gruppe-5-Metalls oder Al oder einem Gemisch daraus gebildet ist.

2. Mikroteilchen nach Anspruch 1, die magnetisch sind, wobei optional der Magnetwerkstoff auf die Oberfläche der Partikel vor der Beschichtung des mindestens einen Übergangsmetalloxids adsorbiert wird.

3. Mikroteilchen nach Anspruch 1 oder Anspruch 2, die porös sind, wobei optional die Mikroteilchen magnetisch sind und wobei der Magnetwerkstoff in den Poren der Partikel vor der Beschichtung des mindestens einen Übergangsmetalloxids aufgebracht wird.

4. Mikroteilchen, wie beansprucht in irgendeinem vorausgehenden Anspruch, die vor der Beschichtung des mindestens einen Übergangsmetalloxids durch eine zusätzliche Polymerüberzugsschicht bedeckt werden.

5. Mikroteilchen wie beansprucht in irgendeinem vorausgehenden Anspruch, deren Durchmesser einen Variationskoeffizienten von nicht mehr als 8 % aufweisen, wobei optional die Mikroteilchen einen Durchmesser von 0,2 bis 10 $\mu$m aufweisen.

6. Mikroteilchen, wie beansprucht in irgendeinem vorausgehenden Anspruch, wobei das Übergangsmetalloxid Titan-, Tantal-, Niob- oder Zirkonoxid ist.

7. Mikroteilchen, wie beansprucht in irgendeinem vorausgehenden Anspruch, die Styrol-Divinylbenzol-Partikel sind.

8. Ein Prozess für die Herstellung von monodispersen Polymer-Mikroteilchen, die wie vorstehend beschrieben einen Variationskoeffizienten (CV) von weniger als 20 % aufweisen und reagierende Polymer-Mikroteilchen umfassen, die Polystyrol oder Polyacrylat mit mindestens einer Übergangsmetallverbindung eines Gruppe-4- oder Gruppe-5-Metalls oder Al oder ein Gemisch davon enthalten, das dazu fähig ist, in ein Oxid umgewandelt zu werden und z. B. aufgrund von Wärme- und/oder Wasserzufuhr die Übergangsmetalloxid-Beschichtung zu bilden.

9. Prozess nach Anspruch 8, wobei vor der Beschichtung der Mikroteilchen die beginnenden Mikroteilchen oberflächenfunktionalisiert werden, und wobei die Mikroteilchen optional oberflächenfunktionalisiert werden, um Amino-, Hydroxyl-, Epoxid-, Carboxy- oder Siloxy-Gruppen zu tragen.

10. Prozess nach Anspruch 8 oder Anspruch 9, wobei die Übergangsmetallverbindung ein Alkoholat ist.

11. Prozess nach Anspruch 10, wobei das Alkoholat ein Tetra- (C1-6) -Zirconium-Alkoholat oder Titan-Alkoholat ist.

12. Prozess, wie beansprucht in irgendeinem der Ansprüche 8 bis 11, der in mindestens zwei Schritten ausgeführt wird, wobei ein erster Schritt das Kontaktieren der Mikroteilchen mit Übergangsmetall in einer wasserfreien Umgebung und ein zweiter Schritt das Hinzufügen von Wasser zum Produkt des ersten Schrittes umfasst.

13. Ein Verfahren für die Isolierung von Phosphoproteinen von einem Muster, das Phosphoproteine enthält, umfassend das Kontaktieren der Probe mit den monodispersen Polymer-Mikroteilchen der Ansprüche 1 bis 7, wobei das Verfahren optional umfasst:

(I) das Bereitstellen einer Probe, die ein oder mehrere Phosphoproteine enthält;
(II) das Mischen der Probe mit monodispersen Polymer-Mikroteilchen;
(III) das Inkubieren der Probe und der Partikel;
(IV) das Sammeln der Partikel, optional in einem Magnetfeld, und das Entfernen des Überstandes; und optional
(V) das Eluieren von einem oder mehreren isolierten Phosphoproteinen.

14. Verfahren nach Anspruch 13, wobei Schritt (V) in der Gegenwart von einem Puffer ausgeführt wird, der Phenyl-Phosphat und Ammoniaklösung enthält.

15. Verfahren nach Anspruch 13 oder Anspruch 14, wobei Schritt (II) in der Gegenwart von einem Puffer ausgeführt wird, der Wasser, Ethanol und Natriumacetate enthält und einen pH-Wert zwischen 3,9 und 4,1 aufweist.

16. Verfahren, wie beansprucht in jedem der Ansprüche 13 bis 15, wobei Schritt (II) ohne 2, 5-Dihydroxybenzoesäure ausgeführt wird.

17. Verwendung der Polymer-Mikroteilchen, wie beansprucht in einem der Ansprüche 1 bis 7, in Phosphoprotein-Isolierung.

18. Ein Verfahren, um Nukleinsäure aus einer Probe zu isolieren, die Nukleinsäure enthält, umfassend das Kontaktieren der Probe mit monodispersen Polymer-Mikroteilchen nach Anspruch 1 bis 7, wobei das Verfahren optional umfasst:

    (I) das Bereitstellen einer Probe, die eine oder mehrere Nukleinsäuren enthält;
    (II) das Mischen der Probe mit übergangsmetalloxidbeschichteten Mikroteilchen, wie vorstehend beschrieben;
    (III) das Inkubieren der Probe und der Partikel;
    (IV) das Sammeln der Partikel, optional in einem Magnetfeld, und das Entfernen des Überstandes; und
    (V) optional das Eluieren von einer oder mehreren isolierten Nukleinsäuren.

19. Verfahren nach Anspruch 18, bei dem der Kontakt zwischen der Nukleinsäure und den Mikroteilchen in Gegenwart von einem chaotropen Puffer stattfindet.

20. Kit für die Isolierung von Nukleinsäuren, umfassend Polymer-Mikroteilchen, wie beschrieben in den Ansprüchen 1 bis 7, und einen chaotropen Puffer.

**Revendications**

1. Microparticules de polymère monodispersées ayant un coefficient de variation (CV) inférieur à 20 % et comprenant un polystyrène ou un polyacrylate, dans lesquelles lesdites particules ont un revêtement formé d'au moins un oxyde de métal de transition d'un métal du groupe 4 ou du groupe 5 ou d'aluminium ou d'un mélange de ces derniers.

2. Microparticules selon la revendication 1, qui sont magnétiques, facultativement là où le matériau magnétique est adsorbé sur la surface des particules avant le revêtement d'au moins un oxyde de métal de transition.

3. Microparticules selon la revendication 1 ou la revendication 2, qui sont poreuses, facultativement dans lesquelles les microparticules sont magnétiques et là où le matériau magnétique est déposé dans les pores des particules avant le revêtement d'au moins un oxyde de métal de transition.

4. Microparticules selon l'une quelconque des revendications précédentes, qui sont recouvertes par une couche polymère supplémentaire avant le revêtement d'au moins un oxyde de métal de transition.

5. Microparticules selon l'une quelconque des revendications précédentes, dont le diamètre a un coefficient de variation inférieur ou égal à 8 %, facultativement dans lesquelles les microparticules ont un diamètre allant de 0,2 à 10 $\mu$m.

6. Microparticules selon l'une quelconque des revendications précédentes, dans lesquelles l'oxyde de métal de transition est l'oxyde de titane, de tantale, de niobium ou de zirconium.

7. Microparticules selon l'une quelconque des revendications précédentes, qui sont des particules de styrène-divinylbenzène.

8. Procédé de préparation de microparticules de polymère monodispersées ayant un coefficient de variation (CV) inférieur à 20 % comme décrit ci-dessus, comprenant des microparticules polymères réactives comprenant un polystyrène ou un polyacrylate avec au moins un composé de métal de transition d'un métal du groupe 4 ou du groupe 5 ou d'aluminium ou d'un mélange de ces derniers qui peut être converti en un oxyde et former un revêtement d'oxyde de métal de transition, par exemple lors de l'application de chaleur et/ou d'eau.

**9.** Procédé selon la revendication 8, dans lequel, avant de revêtir les microparticules, les microparticules de départ sont fonctionnalisées en surface, facultativement dans lequel les microparticules sont fonctionnalisées en surface pour supporter des groupes amino, hydroxyle, époxydes, carboxy ou siloxy.

**10.** Procédé selon la revendication 8 ou la revendication 9, dans lequel le composé de métal de transition est un alcoxyde.

**11.** Procédé selon la revendication 10, dans lequel l'alcoxyde est un tétra-alcoxyde de zirconium ou de titane en C1-C6.

**12.** Procédé selon l'une quelconque des revendication 8 à 11 effectué en au moins deux étapes, une première étape consistant à mettre en contact les microparticules avec un composé de métal de transition dans un milieu anhydre, et une deuxième étape consistant à ajouter de l'eau au produit de la première étape.

**13.** Procédé permettant l'isolement des phosphoprotéines d'un échantillon contenant des phosphoprotéines, ledit procédé consistant à mettre en contact ledit échantillon avec les microparticules de polymère monodispersées selon les revendications 1 à 7, le procédé consistant facultativement à :

(I) fournir un échantillon qui contient une ou plusieurs phosphoprotéines ;
(II) mélanger l'échantillon avec les microparticules de polymère monodispersées ;
(III) incuber l'échantillon et les particules ;
(IV) recueillir les particules, facultativement dans un champ magnétique, et retirer le surnageant ; et facultativement
(V) éluer une ou plusieurs phosphoprotéines isolées.

**14.** Procédé selon la revendication 13, dans lequel l'étape (V) est effectuée en présence d'un tampon comprenant le phosphate de phényle et l'hydroxyde d'ammonium.

**15.** Procédé selon la revendication 13 ou la revendication 14, dans lequel l'étape (II) est effectuée en présence d'un tampon comprenant de l'eau, de l'éthanol et de l'acétate de sodium et ayant un pH compris entre 3,9 et 4,1.

**16.** Procédé selon l'une quelconque des revendications 13 à 15, dans lequel l'étape (II) est effectuée en l'absence d'acide 2, 5-dihydroxybenzoïque.

**17.** Utilisation des microparticules de polymère selon l'une quelconque des revendications 1 à 7 dans un isolement de phosphoprotéines.

**18.** Procédé permettant d'isoler l'acide nucléique d'un échantillon contenant de l'acide nucléique, ledit procédé consistant à mettre en contact ledit échantillon avec des microparticules de polymère monodispersées selon les revendications 1 à 7, le procédé consistant facultativement à :

(I) fournir un échantillon qui contient un ou plusieurs acides nucléiques ;
(II) mélanger l'échantillon avec un oxyde de métal de transition recouvert de microparticules comme décrit ci-dessus ;
(III) incuber l'échantillon et les particules ;
(IV) recueillir les particules, facultativement dans un champ magnétique, et retirer le surnageant ; et
(V) facultativement éluer un ou plusieurs acides nucléiques isolés.

**19.** Procédé selon la revendication 18, dans lequel le contact entre l'acide nucléique et les microparticules se produit en présence d'un tampon chaotropique.

**20.** Trousse pour l'isolement des acides nucléiques comprenant des microparticules de polymère comme décrit dans les revendications 1 à 7 et un tampon chaotropique.

1 2 3 4 5 6 7

Figure 1

Figure 2

1 2 3 4 5 6 7 8 9

Figure 3

Figure 4

Figure 5

Figure 6

Figure 7

Figure 8

Figure 9

Figure 10

Figure 11a

Figure 11b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4654267 A
- US 6383393 B
- US 5057426 A
- US 6914137 B
- WO 05015216 A
- WO 9919375 A
- WO 0061648 A
- WO 0170825 A
- WO 0061647 A
- US 5234809 A
- WO 9112079 A
- US 5705628 A
- US 5898071 A
- WO 9618731 A

### Non-patent literature cited in the description

- *Anal. Chem,* 2005, vol. 77, 5912-5919
- **GUO et al.** *Optical Materials,* 2003, vol. 22, 39-44